Europäisches Patentamt

(19) European Patent Office

Office européen des brevets

(11) Publication number: **0 039 996**
**B1**

(12) # EUROPEAN PATENT SPECIFICATION

(45) Date of publication of patent specification: **02.11.83**

(21) Application number: **81301496.6**

(22) Date of filing: **07.04.81**

(51) Int. Cl.³: **B 01 J 21/00,**
**B 01 J 37/02, C 10 G 3/00,**
**C 07 C 1/20, C 08 F 10/00,**
**C 08 F 4/12**

(54) Alumina and/or silica catalysts supported on microporous glass

(30) Priority: **09.05.80 GB 8015449**

(43) Date of publication of application:
**18.11.81 Bulletin 81/46**

(45) Publication of the grant of the patent:
**02.11.83 Bulletin 83/44**

(84) Designated Contracting States:
**DE FR GB IT NL SE**

(56) References cited:
**GB - A - 922 381**
**GB - A - 1 446 522**
**US - A - 2 835 637**
**US - A - 3 804 647**
**US - A - 3 923 688**

(73) Proprietor: **Coal Industry (Patents) Limited**
**Hobart House Grosvenor Place**
**London SW1X 7AE (GB)**

(72) Inventor: **Robinson, Joseph Gordon**
**Claybrook The Hyde**
**Winchcombe Gloucestershire (GB)**
Inventor: **Barnes, David Ian**
**70 Fiddlers Green Lane**
**Cheltenham Gloucestershire (GB)**
Inventor: **Carswell, Angela Mary**
**21 The Willows**
**Longhope Gloucestershire (GB)**

(74) Representative: **Wood, John Irwin**
**Hobart House Grosvenor Place**
**London SW1X 7AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Alumina and/or silica catalysts supported on microporous glass

This invention concerns improvements in catalysis and provides novel catalysts, their production and catalytic processes utilising such catalysts.

It has previously been proposed to use zeolites, such as the synthetic zeolite ZSM-5, in the conversion of ketones to aromatic hydrocarbons. It has also been proposed, in GB—A—1 446 522, to use a known crystalline alumino-silicate zeolite having a silica/alumina ratio of at least 12 and having pores of about 5 to 10 Å in diameter, to catalyse the conversion of oxygen-containing organic compounds to low molecular weight aromatics. Such zeolites may be natural mineral zeolites or synthetic zeolites having the requisite properties specified in the patent, of which the most important appears to be that of pore size. It is believed that the synthetic zeolite ZSM-5 is formed by the formation of a gel-type precipitate which is crystallised by heating to give a zeolite having an analytical molar ratio of $Na_2O : Al_2O_3 : SiO_2 = 0.33 : 1.00 : 26.3$; the Na cations may be exchanged for H using HCl to give an acidic form of analytical molar ratio of $Na_2O : Al_2O_3 : SiO_2 = 0.022 : 1.00 : 43.6$.

We have now discovered novel catalysts which are not zeolites but have certain similarities in utility thereto while offering a number of advantages.

The present invention provides a catalyst comprising a microporous glass having a deposit on the surface of the pores of an alumina, an alumina/silica or a hydrate thereof, a majority of the pores thus reduced in size being in the size range of 5 to 6 Å (50 to 60 nm).

The invention also provides a method of producing a catalyst according to the invention comprising impregnating a microporous glass with a precursor for an alumina or an alumina/silica in an amount sufficient to give a catalyst having a majority of pores in the size range 5 to 6 Å diameter, and converting the precursor into the desired alumina or alumina/silica or hydrate thereof.

The microporous glasses used in the present invention are generally those having pore sizes in the range 20 to 200 Å and have the necessary structural strength and stability to act as a support under the conditions of use of the catalyst as well as being of chemical stability when the alumina or alumina/silica is deposited thereon. It is preferred that the pores of the glass are substantially uniform in size and shape, so that the catalyst prepared according to the invention also has uniform pores. Preferred mean pore sizes in the glass are in the range 30 to 60 Å (300 to 600 nm). A preferred glass is known as controlled pore glass. Controlled pore glass is commercially available and is in the form of rigid glass beads honeycombed with a multiplicity of pores of very uniform size. It is believed that it is prepared by etching out the borates from a borosilicate glass. Controlled pore glass is available in a variety of pore sizes down to 40 Å and is recommended for use in exclusion chromatography. It is believed that the high degree of uniformity of pore size in the controlled pore glass is maintained in the catalysts of the present invention, leading to very substantial advantages in uniformity of catalytic activity and control of the products. Natural and synthetic zeolites do not exhibit such uniformity of pore size.

The deposit of silica or silica/alumina may be carried out in a single step or by building up two or more layers to the desired pore size. If two or more layers are deposited, they need not be identical in composition, providing the topmost layer is the desired alumina or alumina/silica or hydrate thereof. While the deposition of alumina may be carried out in any desired manner, a preferred method is to absorb aluminium sec-butoxide, which may be in the presence of the solvent such as sec-butanol, on the catalyst support. Any solvent is then driven off and the sec-butoxide is converted to amorphous aluminium oxide hydrate by hydrolysis, for example by exposure to wet steam at 100°C. Preferably the catalyst is then heat treated, e.g. at 400°C, to yield a deposit of partially hydroxylated alumina on the support. Different crystal forms of alumina can be prepared by ageing for different times at different temperatures up to about 600°C. Preferred crystalline forms are boehmite, pseudo-boehmite, bayerite, gibbsite and nordstandite.

If an alumina/silica deposit is desired, the requisite molar ratio of aluminium to silicon has to be provided. A preferred method is to impregnate the support with the previously calculated quantity of silicon tetrachloride in aluminium sec-butoxide solution, and then to hydrolyse and age as described above.

The present invention also includes catalysts as previously described in admixture with or in train with other catalysts. Furthermore, the catalysts according to the invention may be impregnated with a metal or metals having specific catalytic activity, to alter the product slate obtainable and/or to enable conversion to be carried out at lower temperature. Each additional metal is preferably used in amount of up to 10% by weight and is chosen from zinc, chromium, molybdenum, tungsten, cobalt, nickel, platinum and palladium. These metals may be impregnated onto the catalyst of the invention by methods which are known per se for the production of catalysts such as hydrocracking catalysts on alumina supports, involving impregnation by a salt of the metal and conversion by calcining to the corresponding oxide.

Although the action of the small pore catalysts of the invention is not clear, and we do not wish to be limited to any theory, it is believed that the small pores physically restrict the size of molecules formed to those of mononuclear aromatics and lower aliphatics, and that there are significant electrostatic effects in the pores. It is believed, however, that for the conversion of oxygen-containing organics, and analogous compounds such as thiols, by dehydration or decarboxylation, it is advantageous for the surface of the alumina or alumina/silica to be hydroxylated to a suitable extent.

The catalysts according to the invention are especially suitable for use in reactions in which an oxygen-containing organic compound is dehydrated or decarboxylated to a hydrocarbon, operating at a temperature of at least 250°C. Such reactions are conveniently carried out at or close to atmospheric pressure, at a temperature of 250 to 500°C, preferably 300 to 450°C. Suitable starting materials include lower straight or branched chain alcohols, such as methanol, ethanol and isopropanol, carbonyl compounds, including aldehydes, for example acetaldehyde and propionaldehyde, ketones, for example acetone, methyl ethyl ketone and acetophenone, carboxylic acids, anhydrides and esters thereof, for example acetic acid and anhydride, ethyl acetate, methyl formate, butyric and propionic acids, and ethers such as dimethyl ether, diethyl ether, isopropyl ether and n-butyl methyl ether. The starting materials may comprise straight or branched chain aliphatic groups and may comprise substituted or unsubstituted mononuclear aromatic groups. The starting material need not be a single compound but may be a mixture of compounds or a product stream containing impurities, and in particular may be or may include thio compounds analogous to the oxygen-containing compounds mentioned above, and corresponding unsaturated compounds. The starting material may be derived from a fossil fuel, such as coal, by conversion of said fuel to synthesis gas followed by conversion of said synthesis gas to a mixture of oxygen-containing compounds, for instance a mixture comprising methanol and dimethyl ether as the most abundant compounds.

A particularly preferred starting material is methanol, which may be produced from synthesis gas which itself may be the product of gasification of solid carbonaceous matter including coal, lignite, oil shales and tar sands, as well as hydrocarbons of petroleum origin. The invention includes a process for the production of light hydrocarbons from synthesis gas, either via the intermediate methanol or in which there is no separate step of production of methanol. If there is a separate step of production of methanol this may be a methanol synthesis as known in the art; because of the non-specificity of the catalyst of the invention separation of a methanol stream need not be undertaken, and a feed resulting from Fischer-Tropsch or methanol synthesis which contains a variety of oxygen-containing compounds and hydrocarbons may be used. Alternatively, synthesis gas may be fed as or as part of the feed to the catalyst of the invention which catalyst may be a straight catalyst or in admixture or in train with another catalyst which catalyses the conversion of the synthesis gas.

The products obtained by the use of the catalysts of the invention and starting materials as described above are a range of aliphatic and aromatic hydrocarbons and water. Because of the nature of the catalyst, the hydrocarbon products are those of relatively small molecular size and weight while at the same time less valuable light hydrocarbons, more especially methane, are present in relatively small amounts, although olefins such as ethylene and/or propylene are found in commercially significant quantities. The products are therefore valuable chemical feedstocks and may be separated into individual components for use in chemical synthesis or certain products only, such as the olefins and lower alkanes, may be separated since the remaining hydrocarbons form, without further processing, high grade gasoline boiling range liquid.

The invention therefore also provides a process for the preparation of light hydrocarbons which process comprises passing an oxygen-containing organic compound over a catalyst according to the invention at a temperature of at least 250°C. The reaction pressure is not critical and may vary within wide limits from close to 0 to 200 bar, although it is convenient to operate at or close to atmospheric pressure. The invention further provides a process for the preparation of a polyolefin which process comprises passing an oxygen-containing organic compound over a catalyst according to the invention at a temperature of at least 250°C, separating an olefin component from the products thereof and polymerising said olefins under conditions known *per se*. The main olefin components in the products are formed to be ethylene and propylene; these may be polymerised by gaseous or solution polymerisation over a Ziegler-Natta catalyst under known conditions, to yield low-density or high density polyethylene or polypropylene.

The invention will now be described by way of example

## Comparative Example A

A commercially available controlled pore glass (Pierce CPG) which is essentially a silica skeleton having a pore size of 40 ± 1 Å was packed into a stainless steel tube reactor which was heated by electrical resistance windings. When the temperature, as measured by a thermocouple, had stabilised at 400°C, methanol was passed through using a variable positive displacement pump and a micrometer flow control at a liquid hourly space velocity (LHSV) of 0.3.

The effluent from the reactor was collected in traps cooled by ice water and by acetone/solid carbon dioxide, any remaining gaseous material being passed to a gas burette.

The effluent was collected, weighed and analysed. Methanol was recovered in almost 100% yield and no other product was detected.

## Example 1

A weighed mass of controlled pore glass described in Comparative Example 1 was charged into a vessel which was then evacuated and a pre-determined quantity of aluminium sec-butoxide, as a solution in sec-butanol was slowly added from a hypodermic syringe. The amount of aluminium sec-butoxide was sufficient to give a film of amorphous alumina hydrate within the pores to reduce the mean pore diameter to 5—6 Å. The solvent present was driven off by heating the vessel at 50—60°C under reduced pressure and the alkoxide-coated controlled pore glass was exposed to steam at 100°C to hydrolyse the butoxide to amorphous alumina hydrate. A good bond to the controlled pore glass was obtained and studies indicated a good even deposit within the pores.

The prepared catalyst was charged into a stainless steel tube reactor as described in Comparative Example 1, and methanol vapour was fed through at a liquid hourly space velocity (LHSV) of 0.3, operating at a catalyst temperature of 400°C. 100% conversion methanol was achieved. The major product was dimethyl ether in a yield of 60% of theoretical (based on methanol conversion) and gases containing ethylene and propylene, and water.

Operation at higher temperatures, for example at 450°C, also gave 100% conversion of the feed methanol and also the virtual disappearance of dimethyl ether; a mixture of hydrocarbons including benzene, toluene, xylenes, isobutane and $C_9$ and $C_{10}$ aromatics was obtained.

## Example 2

A catalyst was prepared as described in Example 1 above, except that the steam passage over the coated controlled pore glass was continued until the alkoxide had been converted to the microcrystalline hydrate pseudo-boehmite as demonstrated by X-ray powder diffraction data. Complete conversion of the methanol was achieved at temperatures of 400°C and 450°C, the major products being identified as aromatic hydrocarbons including toluene, benzene, durene, xylene, ethylene and propylene, and water.

## Example 3

A catalyst was prepared as described in Example 2 above except that the steam passage over the coated controlled pore glass was continued until the alkoxide had been converted to the crystalline monohydrate, pseudo-boehmite, as demonstrated by X-ray powder diffraction data. Complete conversion of the methanol was achieved at temperatures of 400°C and 450°C, the major products being toluene, xylene, mesitylene, isobutene, ethylene and propylene and water.

## Example 4

A catalyst was prepared as described in Example 2 but instead of using it directly it was heat-treated at 450°C for 8 hours to yield an oxide (identified as alumina by X-ray diffraction). Thereafter methanol vapour was passed over it as described in Example 1, and complete conversion was achieved at temperatures of 400°C and 450°C to yield, as major products, toluene, durene, isobutene and propylene.

## Example 5

A catalyst was prepared as described in Example 1, but instead of using aluminium sec butoxide solution, a solution of silicon tetrachloride and aluminium sec. butoxide in sec. butanol was used. After hydrolysis the coated controlled pore glass was heat-treated at 600°C for 8 hours to yield a catalyst having a silica/alumina ratio of 15 and a mean pore diameter of 8Å.

The catalyst was effective at 400°C in completely converting methanol to hydrocarbons and water. The principal hydrocarbons were toluene, xylenes, $C_9$ aromatics and $C_{10}$ aromatics and $C_2$, $C_3$, $C_4$ and $C_5$ aliphatics.

## Example 6

The catalysts of above Examples 1 to 3 are used to convert propionaldehyde, acetal, acetic acid and butyl acrylate instead of methanol. Complete or virtually complete conversion of the starting material is achieved, and the products are branched chain alkyls and substituted benzenes boiling in the gasoline range. Research Octane Numbers of about 70 are obtained without further processing thereof.

## Claims

1. A catalyst comprising a microporous glass having a deposit on the surface of the pores of an alumina, alumina/silica or a hydrate thereof, a majority of the pores thus reduced in size being in the size range 5 to 6 Å (50 to 60 nm) diameter.

2. A catalyst according to claim 1, wherein the microporous glass is a controlled pore glass.

3. A catalyst according to claim 2, wherein the controlled pore glass has a mean pore size in the range 30 to 60 Å (300 to 600 nm).

4. A catalyst according to any one of the preceding claims, wherein the alumina, alumina/silica or hydrate thereof carries one or more metals selected from zinc, chromium, molybdenum, tungsten, cobalt, nickel, platinum and palladium.

5. A catalyst according to claim 4, wherein the or each metal is present in an amount of up to 10% by weight.

6. A catalyst according to any one of the preceding claims, wherein alumina is present in a crystalline form selected from boehmite, pseudo-boehmite, bayerite, gibbsite and nordstandite.

7. A method of producing a catalyst comprising impregnating a microporous glass with a precursor for an alumina or an alumina/silica in an amount sufficient to give a catalyst having a majority of pores in the size range 50 to 60 nm diameter, and converting the precursor into the desired alumina, alumina/silica or hydrate thereof.

8. A method according to claim 7, wherein the microporous glass is a controlled pore glass.

9. A method according to claim 7 or 8, wherein the microporous glass has, before impregnation, a mean pore size of from 300 to 600 nm.

10. A method according to any one of claims 7 to 9, wherein the precursor for alumina is aluminium sec-butoxide.

11. A method according to claim 10, wherein the aluminium sec-butoxide is converted to aluminium oxide hydrate by hydrolysis.

12. A method according to claim 11, wherein the aluminium oxide hydrate is heat treated to yield a desired form of alumina or alumina hydrate.

13. A method according to any one of claims 7 to 12, wherein the precursor for alumina/silica or hydrate thereof is silicon tetrachloride in aluminium sec-butoxide solution.

14. A method according to any one of claims 7 to 13, wherein the alumina, alumina/silica or hydrate thereof is impregnated with one or more metals selected from zinc, chromium, molybdenum, tungsten, cobalt, nickel, platinum and palladium.

15. A process for the preparation of light hydrocarbons which process comprises passing an oxygen-containing organic compound over a catalyst according to any one of claims 1 to 6 at a temperature of at least 250°C.

16. A process according to claim 15, wherein the oxygen-containing organic compound is an alcohol.

17. A process according to claim 16, wherein the alcohol is methanol, ethanol or isopropanol.

18. A process according to claim 15, wherein the oxygen-containing organic compound is a carbonyl compound.

19. A process according to claim 18, wherein the carbonyl compound is acetaldehyde, propionaldehyde, acetone, methyl ethyl ketone, acetophenone, acetic acid or anhydride, ethyl acetate, methyl formate, butyric acid or propionic acid.

20. A process according to claim 15, wherein the oxygen-containing organic compound is an ether.

21. A process according to claim 20, wherein the ether is dimethyl ether, diethyl ether, isopropyl ether or n-butyl methyl ether.

22. A process according to any one of claims 15 to 21, wherein a mixture of oxygen-containing organic compounds is passed over the catalyst.

23. A process according to claim 22, wherein said mixture is derived from a fossil fuel by conversion of said fuel to synthesis gas followed by conversion of said synthesis gas to the mixture of oxygen-containing organic compounds.

24. A process according to claim 23, wherein the mixture comprises methanol and dimethyl ether as the most abundant compounds.

25. A process according to claim 23 or 24, wherein the fossil fuel is coal.

26. A process as described in any one of claims 15 to 22, wherein an analogous thio compound is used instead of the oxygen-containing compound.

27. A process for the preparation of light hydrocarbons which process comprises passing synthesis gas over a catalyst according to any one of claims 1 to 6 used alone or in admixture or in train with a conversion catalyst, at a temperature of at least 250°C.

28. A process for the production of a polyolefin which process comprises passing an oxygen-containing organic compound over a catalyst according to any one of claims 1 to 6 at a temperature of at least 250°C, separating an olefin component from the products thereof and polymerising said olefin.

29. A process according to claim 28, wherein the olefin is ethylene or propylene.

**Revendications**

1. Catalyseur comprenant un verre microporeux présentant, à la surface des pores, un dépôt d'une alumine, d'une alumine/silice ou d'un de leur hydrate, une majorité des pores de dimensions ainsi réduites se situant dans la gamme des dimensions de 5 à 6 Å (50 à 60 nm de diamètre).

2. Catalyseur selon la revendication 1, dans lequel le verre microporeux est un verre à pores réglés.

3. Catalyseur selon la revendication 2, dans lequel le verre à pores réglés présente une dimension moyenne des pores comprise entre 30 et 60 Å (300 à 600 nm).

4. Catalyseur selon l'une quelconque des revendications précédentes, dans lequel l'alumine, l'alumine/silice ou leurs hydrates portent un ou plusieurs métaux choisis parmi le zinc, le chrome, le molybdène, le tungstène, le cobalt, le nickel, le platine et le palladium.

5. Catalyseur selon la revendication 4, dans lequel le ou chaque métal est présent en une quantité dont le maximum est de 10% en poids.

6. Catalyseur selon l'une quelconque des revendications précédentes, dans lequel l'alumine est présente sous forme cristalline choisie parmi le boehmite, la pseudoboehmite, la bayerite, la gibbsite et la nordstandite.

7. Procédé de production d'un catalyseur comprenant l'imprégnation d'un verre microporeux à l'aide d'un précurseur d'une alumine ou d'une alumine/silice en une quantité suffisante pour donner un catalyseur ayant une majorité de pores se situant dans la gamme des dimensions de 50 à 60 nm de diamètre, et la transformation du précurseur en l'alumine, l'alumine/silice ou son hydrate voulu.

8. Procédé selon la revendication 7, dans lequel le verre microporeux est un verre à pores réglés.

9. Procédé selon la revendication 7 ou 8, dans lequel le verre microporeux possède, avant imprégnation, une dimension moyenne des pores comprise entre 300 et 600 nm.

10. Procédé selon l'une quelconque des revendications 7 à 9, dans lequel le précurseur de l'alumine est du butylate secondaire d'aluminium.

11. Procédé selon la revendication 10, dans lequel le butylate secondaire d'aluminium est transformé par hydrolyse en oxyde d'aluminium hydraté.

12. Procédé selon la revendication 11, dans lequel l'oxyde d'aluminium hydraté est traité par chauffage pour donner une forme voulue d'alumine ou d'hydrate d'alumine.

13. Procédé selon l'une quelconque des revendications 7 à 12, dans lequel le précurseur de l'alumine/silice ou de son hydrate est une solution de tétrachlorure de silicium dans du butylate secondaire d'aluminium.

14. Procédé selon l'une quelconque des revendications 7 à 13, dans lequel on imprègne l'alumine, l'alumine/silice ou son hydrate à l'aide d'un ou plusieurs métaux choisis parmi le zinc, le chrome, le molybdène, le tungstène, le cobalt, le nickel, le platine et le palladium.

15. Procédé de préparation d'hydrocarbures légers, qui comprend le passage d'un composé organique contenant de l'oxygène sur un catalyseur selon l'une quelconque des revendications 1 à 6, à une température d'au moins 250°C.

16. Procédé selon la revendication 15, dans lequel le composé organique contenant de l'oxygène est un alcool.

17. Procédé selon la revendication 16, dans lequel l'alcool est le méthanol, l'éthanol ou l'isopropanol.

18. Procédé selon la revendication 15, dans lequel le composé organique contenant de l'oxygène est un composé carbonylé.

19. Procédé selon la revendication 18, dans lequel le composé carbonylé est l'acétaldéhyde, le propionaldéhyde, l'acétone, la méthyl éthyl cétone, l'acétophénone, l'acide ou l'anhydride acétique, l'acétate d'éthyle, le formiate de méthyle, l'acide butyrique ou l'acide propionique.

20. Procédé selon la revendication 15, dans lequel le composé organique contenant de l'oxygène est un étheroxyde.

21. Procédé selon la revendication 20, dans lequel l'éther-oxyde est l'éther-oxyde de diméthyle, l'éther-oxyde de diéthyle, l'éther-oxyde d'isopropyle ou l'éther-oxyde de butyle normal et de méthyle.

22. Procédé selon l'une quelconque des revendications 15 à 21, dans lequel on fait passer sur le catalyseur un mélange de composés organiques contenant de l'oxygène.

23. Procédé selon la revendication 22, dans lequel ledit mélange provient d'un combustible fossile par transformation dudit combustible en du gaz de synthèse suivie d'une transformation dudit gas de synthèse en le mélange de composés organiques contenant de l'oxygène.

24. Procédé selon la revendication 23, dans lequel le mélange comprend du méthanol et de l'éther oxyde de diméthyle comme composés les plus abondants.

25. Procédé selon la revendication 23 ou 24, dans lequel le combustible fossile est du charbon.

26. Procédé selon l'une quelconque des revendications 15 à 22, dans lequel on utilise un composé thio analogue au lieu du composé contenant de l'oxygène.

27. Procédé pour la préparation d'hydrocarbures légers, ce procédé comprenant le passage du gaz de synthèse sur un catyalyseur selon l'une quelconque des revendications 1 à 6, utilisé seul ou en mélange ou en série avec un catalyseur de transformation, à une température d'au moins 250°C.

28. Procédé de production d'une polyoléfine, ce procédé comprenant le passage d'un composé organique contenant de l'oxygène sur un catalyseur selon l'une quelconque des revendications 1 à 6 à une température d'au moins 250°C, la séparation d'un constituant oléfinique d'avec les produits de la réaction et la polymérisation de ladite oléfine.

29. Procédé selon la revendication 28, dans lequel l'oléfine est l'éthylène ou le propylène.

## Patentansprüche

1. Katalysator, gekennzeichnet durch mikroporöses Glas mit einer Ablagerung aus Aluminiumoxid, Aluminiumoxid/Siliciumoxid oder einem Hydrat davon auf der Porenoberfläche, wobei der größte Teil der in ihrer Größe so verminderten Poren einen Durchmesser zwischen 50 und 60 nm aufweist.

2. Katalysator nach Anspruch 1, dadurch gekennzeichnet, daß das mikroporöse Glas ein kontrolliertes Porenglas ist.

3. Katalysator nach Anspruch 2, dadurch gekennzeichnet, daß das Porenglas eine mittlere Porengröße im Bereich zwischen 300 und 600 nm aufweist.

4. Katalysator nach einem der vorstehenden Ansprüche, dadurch gekennzeichnet, daß das Aluminiumoxid, Aluminiumoxid/Siliciumoxid oder das Hydrat davon eines oder mehrere der Metalle: Zink, Chrom, Molybdän, Wolfram, Kobalt, Nickel, Platin und Palladium enthält.

5. Katalysator nach Anspruch 4, dadurch gekennzeichnet, daß das oder jedes Metall in einer Menge bis zu 10 Gewichtsprozent vorhanden ist.

6. Katalysator nach einem der vorstehenden Ansprüche, dadurch gekennzeichnet, daß das Aluminiumoxid in kristallisierter Form als Boehmit, Pseudoboehmit, Bayerit, Gibbsit oder Nordstandit vorliegt.

7. Verfahren zur Herstellung eines Katalysators nach einem der Ansprüche 1 bis 6, dadurch gekennzeichnet, daß das mikroporöse Glas mit einer Aluminiumoxid oder Aluminiumoxid/Siliciumoxid bildenden Zusammensetzung in einer Menge imprägniert wird, die einen Katalysator ergibt, dessen Poren größtenteils einen Durchmesser von 50 bis 60 nm aufweisen, wobei die Zusammensetzung zu dem gewünschten Aluminiumoxid, Aluminiumoxid/Siliciumoxid oder dem Hydrat davon umgesetzt wird.

8. Verfahren nach Anspruch 7, dadurch gekennzeichnet, daß das mikroporöse Glas ein kontrolliertes Porenglas ist.

9. Verfahren nach Anspruch 7 und 8, dadurch gekennzeichnet, daß das mikroporöse Glas vor dem Imprägnieren eine mittlere Porengröße von 300 bis 600 nm aufweist.

10. Verfahren nach einem der Ansprüche 7 bis 9, dadurch gekennzeichnet, daß die Aluminiumoxid bildende Zusammensetzung Aluminium-sek.-Butoxid ist.

11. Verfahren nach Anspruch 10, dadurch gekennzeichnet, daß das Aluminium-sek.-Butoxid in Aluminiumoxidhydrat durch Hydrolyse umgewandelt wird.

12. Verfahren nach Anspruch 11, dadurch gekennzeichnet, daß das Aluminiumoxidhydrat wärmebehandelt wird, um die gewünschten Formen des Aluminiumoxids oder Aluminiumoxidhydrats zu erhalten.

13. Verfahren nach einem der Ansprüche 7 bis 12, dadurch gekennzeichnet, daß die das Aluminiumoxid/Siliciumoxid oder dessen Hydrat bildende Zusammensetzung Siliciumtetrachlorid in einer Lösung von Aluminium-sek.-Butoxid ist.

14. Verfahren nach einem der Ansprüche 7 bis 13, dadurch gekennzeichnet, daß das Aluminiumoxid, Aluminiumoxid/Siliciumoxid oder das Hydrat davon zusammen mit einem oder mehreren Metallen der Gruppe: Zink, Chrom, Molybdän, Wolfram, Kobalt, Nickel, Platin und Palladium imprägniert wird.

15. Verwendung des Katalysators nach einem der Ansprüche 1 bis 6 zur Herstellung von leichten Kohlenwasserstoffen, wobei eine sauerstoffhaltige organische Verbindung über den Katalysator bei einer Temperatur von mindestens 250°C geleitet wird.

16. Verfahren nach Anspruch 15, dadurch gekennzeichnet, daß die sauerstoffhaltige organische Verbindung ein Alkohol ist.

17. Verfahren nach Anspruch 16, dadurch gekennzeichnet, daß der Alkohol Methanol, Athanol oder Isopropanol ist.

18. Verfahren nach Anspruch 15, dadurch gekennzeichnet, daß die sauerstoffhaltige organische Verbindung eine Carbonylverbindung ist.

19. Verfahren nach Anspruch 18, dadurch gekennzeichnet, daß die Carbonylverbindung Acetaldehyd, Propionaldehyd, Aceton, Methyläthylketon, Acetophenon, Essigsäure oder Essigsäureanhydrid, Äthylacetat, Ameisensäuremethylester, Buttersäure oder Propionsäure ist.

20. Verfahren nach Anspruch 15, dadurch gekennzeichnet, daß die sauerstoffhaltige organische Verbindung ein Äther ist.

21. Verfahren nach Anspruch 20, dadurch gekennzeichnet, daß der Äther Dimethyläther, Diethyläther, Isopropyläther oder n-Butylmethyläther ist.

22. Verfahren nach einem der Ansprüche 15 bis 21, dadurch gekennzeichnet, daß ein Gemisch aus sauerstoffhaltigen organischen Verbindungen über den Katalysator geleitet wird.

23. Verfahren nach Anspruch 22, dadurch gekennzeichnet, daß das Gemisch herrührt von einem fossilen Brennstoff, wobei der Brennstoff in Sythesegas umgewandelt worden ist, gefolgt von einer Umwandlung des Synthesegases zu einem Gemisch sauerstoffhaltiger organischer Verbindungen.

24. Verfahren nach Anspruch 23, dadurch gekennzeichnet, daß das Gemisch Methanol und Dimethyläther als die häufigsten Verbindungen umfaßt.

25. Verfahren nach Anspruch 23 oder 24, dadurch gekennzeichnet, daß der fossile Brennstoff Kohle ist.

26. Verfahren nach einem der Ansprüche 15 bis 22, dadurch gekennzeichnet, daß eine analoge Thioverbindung anstelle der sauerstoffhaltigen Verbindung verwendet wird.

27. Verwendung des Katalysators nach einem der Ansprüche 1 bis 26 zur Herstellung leichter Kohlenwasserstoffe, wobei Synthesegas über den Katalysator, der entweder allein oder in einem Gemisch mit oder nach einem Umwandlungskatalysator verwendet wird, bei einer Temperatur von mindestens 250°C geleitet wird.

28. Verwendung des Katalysators nach einem der Ansprüche 1 bis 6 zur Herstellung eines Polyolefins, wobei die sauerstoffhaltige organische Verbindung über den Katalysator bei einer Temperatur von mindestens 250°C geleitet, die Olefinkomponente von den Produkten abgetrennt und das Olefin polymerisiert wird.

29. Verfahren nach Anspruch 28, dadurch gekennzeichnet, daß des Olefin Athylen oder Propylen ist.